Europäisches Patentamt

European Patent Office

Office européen des brevets

(11) Publication number: **0 519 250 A2**

# EUROPEAN PATENT APPLICATION

(21) Application number: **92109083.3**

(22) Date of filing: **29.05.92**

(51) Int. Cl.5: **G01N 33/543**, //G01N33/553, G01N27/22,G01N22/00, G01N33/76

(30) Priority: **10.06.91 US 713432**

(43) Date of publication of application: **23.12.92 Bulletin 92/52**

(84) Designated Contracting States:
**AT BE CH DE DK ES FR GB GR IT LI LU NL SE**

(71) Applicant: **MILES INC.**
**One Mellon Center 500 Grant Str.**
**Pittsburgh, PA 15219-2502(US)**

(72) Inventor: **Buckler, Robert T.**
**23348 May Street**
**Edwardsburg, MI 49112(US)**
Inventor: **Vogelhut, Paul O.**
**12497 Dragoon Trail**
**Mishawaka, IN 46544(US)**

(74) Representative: **Dänner, Klaus, Dr. et al**
**c/o Bayer AG Konzernverwaltung RP**
**Patentabteilung**
**W-5090 Leverkusen 1 Bayerwerk(DE)**

(54) Microparticle-labeled binding assay analyzed by microwave spectroscopy.

(57) A method for determining a ligand in a test sample wherein a liquid test mixture is formed in which microparticles, e.g., metal sol particles, are aggregated as a direct or inverse function of the presence or amount of said ligand in said sample, the microparticles having an effective dielectric constant that is significantly different from the dielectric constant of the test mixture in the absence of ligand in the test sample, and any change in dielectric constant of the test mixture in the microwave region is measured by means of microwave spectroscopy. Aggregation can occur in a liquid solution or suspension or at the surface of a solid substrate. Changes in the dielectric constant of the test mixture is preferably measured with a microwave stripline resonance device. The method can be used to perform a variety of binding assays, including immunoassays and nucleic acid hybridization assays.

## BACKGROUND OF THE INVENTION

The present invention relates to a method for determining a ligand in a test sample in which the test response is measured by electronic means. In particular, the present method relates to the determination of a ligand by means of a binding assay involving reagent coated microparticles, and measuring the effect of agglutination of such microparticles on the dielectric properties of the test mixture.

Binding assays are generally understood in the art to involve the determination of a substance of interest (the ligand) by binding with a counterpart substance (a binding partner). Commonly used binding assays include immunoassays and nucleic acid hybridization assays. Such assays are based on the highly specific and sensitive binding interaction between antigens and hapten with antibodies, or between complementary strands of oligonucleotides or polynucleotides. Such assays have proven to be highly powerful analytical tools, particularly in the field of medical diagnostics.

There is a continuing need to improve the analytical performance, e.g., sensitivity, and ease of use of binding assays. Typical commercial assays involve the generation of an optical or radiometric signal which require a spectrophotometer or scintillation counter for quantification. Such approaches have evident limitations and disadvantages. The radiometric approach requires the user to deal with harmful radioactivity, both in terms of exposure during use and in terms of waste disposal, and the instrumentation is quite expensive and bulky. Spectrophotometric methods are limited in their sensitivity and are subject to a variety of interferences from organic materials such as proteins and cellular debris present in most test samples of interest, e.g., urine, serum, or plasma.

As a result of these and other limitations, a number of alternatives have been investigated. Because of the potential for miniaturization and improved analytical performance, a number of attempts have been made to design and develop binding assays in which the assay response is measured by electrochemical or electronic means. To the present, however, such approaches have met with limited commercial success.

U.S. Patent No. 4,219,335 relates to binding assays employing microparticle reagents and measurement of effects on electrical reactance, preferably effects on magnetic fields, at a surface. U.S. Patent No. 4,794,089 describes a method for detecting the effect of specific binding interactions between microparticle borne reagents and binding partners coated onto an electrode surface by measuring electrical conductivity. German OLS 3,802,150 describes means for controlling the dielectric constant of a nonconductive medium such as a liquid by inclusion of materials including microparticles having predetermined properties. Chen and others have investigated the fundamental effects of microparticle aggregation on the optical properties of a medium [Chen et al, AIP Conf. Proc., 160 (Adv. Laser Sci - 2) (1987); Chen et al, Phys. Rev. B, 37(10): 5232 (1988); Devaty and Sievers, Phys. Rev. Lett., 52: 1344 (1984)].

## SUMMARY OF THE INVENTION

The present invention provides a method for determining a ligand in a test sample, comprising the steps of:

(a) forming a liquid test mixture in which microparticles are aggregated as a direct or inverse function of the presence or amount of said ligand in said test sample, the microparticles having an effective dielectric constant that is significantly different from the dielectric constant of the test mixture in the absence of ligand in the test sample, and

(b) measuring any change in a dielectric property of the test mixture in the microwave region.

The microparticles will preferably have an average diameter of between about 20 and 80 nm, will be composed of a metal sol, such as a gold sol, and will normally be coated with ligand or a binding analog or binding partner thereof. Preferably, the dielectric constant of the test mixture is measured by means of microwave resonance spectroscopy, most preferably with a microwave stripline resonance device.

The microparticle aggregation, in general, can occur in a liquid solution or suspension, or at the surface of a solid substrate. Where the aggregation occurs in a solution or suspension, the method preferably comprises the steps of (a) forming a liquid test mixture containing said test sample and microparticles which are coated with said ligand or a binding analog or a binding partner thereof, and if said microparticles are coated with said ligand or binding analog thereof, then the liquid mixture is formed to additionally contain a diffusible form of a binding partner of the ligand, the coated microparticles having an effective dielectric constant that is significantly different from the dielectric constant of the test mixture in the absence of ligand in the test sample, and (b) measuring any change in dielectric constant of the test mixture in the microwave region as a function of the amount or presence of ligand in the test mixture.

Where a solid state detection device is used, the method preferably comprises the steps of (a) forming a liquid test mixture containing said test sample and microparticles which are coated with said ligand or a binding analog or a binding partner thereof, said test mixture being in contact with a solid substrate to which is bound a binding partner of said ligand, the coated microparticles having an effective dielectric constant that is significantly different from the dielectric constant of the test mixture in the absence of ligand in the test sample, and (b) measuring any change in dielectric constant of the test mixture in the microwave region at the surface of said solid substrate as a function of the amount or presence of ligand in the test mixture by means of microwave resonance spectroscopy. The solid substrate comprises a microwave stripline element. Optionally, the liquid test mixture is removed from contact with the solid substrate prior to the measurement step, and preferably the substrate is washed after removal of the test mixture therefrom.

The present method can be applied to a wide range of binding assays, particularly immunoassays and nucleic acid hybridization assays, to determine a wide variety of ligands. Such ligand analyte can be, among others, an antigen or hapten; an oligonucleotide or a polynucleotide; or a hormone, metabolite, or drug; wherein said binding partner is, respectively, an antibody or a fragment thereof; a complementary oligonucleotide or polynucleotide; or a receptor.

Several key advantages of the present invention are apparent. Sensitivity - theoretical calculations indicate that the detection of microparticle aggregation by microwave resonance spectroscopy is several orders of magnitude more sensitive than is achievable by conventional optical spectroscopy. Miniaturization - the microwave resonator circuit for detecting microparticles can be reduced to the size of a printed circuit less than one inch square and a few thousands of an inch thick. Only a few microliters of a liquid test sample or mixture are needed to cover the sensing area of such a circuit. Miniaturization of the detection system to this degree provides considerable economy and convenience of use. In addition, the favorable ratio of sample volume to detection surface area enables realization of rapid test kinetics and short assay times. Elimination of interferences - common organic substances which interfere in optical methods are transparent in the microwave region of the spectrum. Cellular debris, proteins, colored material such as from hemolysis of a blood sample will not interfere with the detection of microparticle aggregation. Precision - test device configurations are compatible with means for delivering precise volumes of a liquid test sample or mixture such as those elements known as capillary gap elements.

BRIEF DESCRIPTION OF THE DRAWINGS

Fig. 1 is a top view of a typical resonance circuit that is useful in the present invention (details are provided in Example 1 - the dimensions in the drawings are approximately 4x actual size).

Fig. 2 is a cross-sectional view of the circuit shown in Fig. 1.

Fig. 3 is a graph showing the effect of aggregation of microparticles on optical absorbance over time. The microparticles were gold sol coated with human chorionic gonadotropin (hCG) and aggregation was initiated by addition of antibody against hCG.

Figs. 4 and 5 are graphs showing the effect of aggregation of microparticles on the frequency of resonance in two different microwave resonance circuits. The microparticles and aggregation initiator were the same as in the experiment whose results are depicted in Fig. 3.

Fig. 6 is a graph showing the effect of microparticle aggregation on a resonant circuit which controls the frequency of oscillation of an active oscillator ("DRO" stands for dielectric resonance oscillator).

DESCRIPTION OF THE PREFERRED EMBODIMENTS

In the present method, microparticles coated with ligand or binding partner are used to provide information about a ligand in a test medium by forming aggregates of such particles as a function of time. The permittivity of the aggregates differs from that of a homogeneous solution (dispersion) because of the close proximity of the particles to each other which induce dipole interaction and multipole distortion of the externally applied electric fields.

When the microparticles are considered as conducting particles of small size, their finite conductivity value has to be accounted for which depends on their size. As is known, the conductivity of metallic particles decreases proportional to the cube of their diameter by eight orders of magnitude from good conductors to insulators. The effect of this finite and size dependent conductivity is to provide a mechanism for the loss of incident electromagnetic energy. The frequency at which the maximum loss occurs depends directly on the particle conductivity, and inversely on the particle density through its permittivity and the permittivity of the medium.

By choosing for the present particle size the value 40 nm, for the permittivity of a typical microparticle (e.g., a gold sol) 1.5, for the medium 80, one finds the frequency of maximum change due to aggregation to be 1 Gigahertz (GHz). A microwave resonance circuit is designed to measure the time dependent changes in permittivity of the test medium.

This measurement of permittivity of the test specimen can be accomplished in many ways which are detailed in books on the subject. Microwave stripline circuits can be used advantageously since they are very sensitive to small changes and easily instrumented in the chosen frequency range.

The most sensitive permittivity measurement and the one using the least quantity of material would be a resonant cavity method of measurement. At this frequency such a cavity can be bulky and inconvenient and therefore stripline technology is preferred where the conductor patterns guiding the electromagnetic waves are deposited on dielectric substrate material, allowing open access for a test specimen in a critical region of the circuit. A resonant circuit can be made from silver paste fired on a dielectric substrate containing a small gap insulating region between conductors onto which the test specimen is deposited. A wall of epoxy around this region keeps the sample in place during the measurement.

In a preferred embodiment, this resonant circuit is measured on an HP Network analyzer equipped with an S-parameter test set. The resonant frequency and Q of the circuit are measured at the beginning of the test, when the specimen is applied. The test is then repeated periodically and the changes as a function of time are recorded. Depending on the type of circuit and the placement of the specimen, a signal is observed where the permittivity changes in the sample are related to changes in the resonant frequency and Q of the circuit. The test sample can be measured by a reference method such as a light scattering apparatus as a function of time and the frequency changes of the resonant circuit calibrated to mean diameter changes of the aggregating particle clusters.

The formation of clusters by aggregation of particles on the clusters themselves is the main process in many phenomena of practical importance in physics, chemistry, biology, medicine, and engineering. A widely used method in describing the dynamics of such systems is the determination of the cluster size distribution function. This quantity describes the time dependence of a number of clusters of a particular mass and can be measured experimentally by methods derived from an understanding of light scattering processes.

If the substance to be tested is to be reacted with a test material in order to form clusters that can be detected, other interfering substances which might generate erroneous signals have to be accounted for. If the test substance contains cellular matter, large proteins, or other macromolecules, as might be the case in whole blood or serum, these light scattering methods are severely restricted in their application as detection methods for cluster size determination. A physical quantity which can be measured in a test specimen that is related to aggregation is electrical polarizability. The only requirement is that the reacting species distinguish themselves from the surrounding medium by virtue of their electrical properties.

The physical quantity which is monitored in the present invention is the polarizability of the test material. As has been known in the development of artificial dielectrics, the dielectric properties of a material can be drastically changed by including smaller particles or objects within the supporting medium in various densities. [Brown, J. and Jackson, W., "The properties of artificial dielectrics at centimetre wavelengths", Proc. I.E.E. 1955, 102 ptIII, p. 11 and El-Kharadly, M.M.Z., and Jackson, W., "The properties of artificial dielectrics comprising arrays of conducting elements", Proc. I.E.E. 1953, 100, ptIII, p. 199]. In the present invention we again create an artificial dielectric and monitor a chemical reaction as a function of time. During the cluster formation, the local density of the material changes and thereby the polarizability of the material.

Only for extremely dilute solutions is it permissible to assume that the electric field acting on each individual element in the presence of the others remains equal to the externally applied field. When the density of particles increases, account must be taken of the interaction between the dipoles. The first correction may be expressed by a dipole interaction field acting in the same direction as the applied field. As the elements of the array are brought into closer and closer proximity, it becomes increasingly inadequate to consider them as simple dipoles. The resulting distortion of the charge distribution may be allowed for by associating with each particle higher order multipoles, the fields due to which decrease much more rapidly with distance than does the dipolar field.

As an analytically solvable example, one may consider a cubical array of spheres (metallic and perfectly conducting) of diameter d and arranged in a lattice of spacing a. When the spheres are uniformly arranged and distributed throughout the volume, the ratio of diameter to spacing may be given as 0.1 for example. The appropriate correction to the dielectric constant neglecting dipolar interaction would be very small, proportional to the cube power of the ratio (d/a).

In the extreme opposite case, rather than being homogeneously distributed throughout the volume of solution, the spheres could form a close packed cubic array containing the same number but occupying a much smaller volume; in this case, higher order multipoles have to be corrected for. The correction factor for that section of the spacing equals the diameter.

Thus, a cube of dimensions 10 micron on a side contains 1000 particles when the overall density is given as $10^{12}$ particles/mL. If the particle diameter is 100 nm and the interparticle distance is 1 micron or 10 x the particle diameter the ratio d/a = 0.1. This would be the case for a relatively homogeneous distribution of particles within the volume.

The case of an extreme clumping can be simulated by aggregation of all 1000 particles into one corner of a cube of the same dimensions. The total concentration stays identical to the one above, but within the aggregate the interparticle dimension is now equal to the diameter and d/a = 1.0. Under these conditions the new permittivity is given by six times the above value. The correction value is derived from the altered polarizability of the particles. It has to be remembered that this type of aggregation into a cubic array in one corner of the total volume is an extreme case and only useful for illustration of the principle involved.

The dynamics of cluster growths follows very particular laws which have been described. [Lin, M.Y., Lindsay, H.M., Weitz, D.A., Ball, R.C., Klein, R., Meakin, P., "Universality in colloid aggregation", Nature, 339:360-362 (1989)]. The resulting cluster densities and therefore d/a values would be less than 1.0 and depend in their solution on parameters peculiar to the reacting system. Two distinct, limiting regimes of irreversible aggregation have been identified. Diffusion limited aggregation occurs when there is negligible repulsive force between the particles, so that the aggregation rate is limited solely by the time taken for clusters to encounter each other by diffusion. Reaction-limited aggregation occurs when there is still a substantial, but not insurmountable, repulsive force between the particles so that the aggregation rate is limited by the time taken for two cluster to overcome this repulsive barrier by thermal activation.

If the clusters undergo considerable restructuring during aggregation as may be the case for biological molecules, the fractal dimension may be considerably higher than for rigidly assembled units. For the case of the present invention, the aggregation kinetics follow a power-law resulting in a straight line on a double logarithmic plot of average cluster diameter versus time.

The method of the present invention can be used in the qualitative or quantitative determination of a wide variety of substances of analytical interest in a wide variety of test samples. Essentially any form of material can serve as a test sample provided that it is a liquid, can be taken up into a liquid form such as by extraction, dissolution, suspension, or the like, or can be added to a liquid test mixture. Normally, the test sample will be liquid in nature, and will often be a biological fluid such as blood, serum, plasma, urine, cerebral fluid, spinal fluid, saliva, swab extracts, and so forth. Non-biological specimens can also be processed such as industrial effluent, food, and samples taken from the environment such as soil extracts or river or lake water. A liquid test mixture can also result from sample pretreatment such as dilution, filtration, concentration, chemical treatment, and so forth. These are all matters within the ordinary skill in the art.

The present method can be applied to the determination of a variety of substances (analytes). Such substances are referred to herein as ligands on the basis that they possess a specific binding interaction with a counterpart substance or binding partner. The ligand will normally be a peptide, polypeptide, protein, carbohydrate, glycoprotein, steroid, nucleic acid, or other organic entity for which a binding partner exists or can be synthesized or otherwise generated. In functional terms, the ligand and its binding partner will usually be selected from the group comprising antigens, haptens, antibodies, complementary oligonucleotides or polynucleotides, hormones, vitamins, metabolites, drugs, and receptors. Typically, the ligand will be an antigen or hapten; an oligonucleotide or a polynucleotide; or a hormone, metabolite or drug; and its binding partner will be, respectively, an antibody or fragment thereof; a complementary oligonucleotide or polynucleotide; or a receptor. Commonly, the ligand will be an antigen (such as an antigenic polypeptide or protein of molecular weight between about 1,000 and about 10,000,000) or a hapten having a molecular weight of at least about 100 and usually less than 1500; and the binding partner will be a corresponding antibody or a fragment thereof such as Fab or Fab'.

Representative polypeptide analytes are angiotensin I and II, C-peptide, oxytocin, vasopressin, neurophysin, gastrin, secretin, bradykinin, and glucagon.

Representative protein analytes include the classes of protamines, mucoproteins, glycoproteins, globulins, albumins, scleroproteins, phosphoproteins, histones, lipoproteins, including, but not intended to be limited to, apolipoproteins such as apolipoprotein-AI and apolipoprotein-B100, chromoproteins, and nucleoproteins. Examples of specific proteins are prealbumin, $\alpha$-lipoproteins, human serum albumin, $\alpha$-acid glycoprotein, $\alpha_1$-antitrypsin, $\alpha_1$-glycoprotein, transcortin, thyroxine binding globulin, haptoglobin, hemoglobin, glycated peptide sequence in the beta-subunit of human hemoglobin, myoglobulin, ceruloplasmin, $\alpha_2$-macroglobulin, $\beta$-lipoprotein, erythopoietin, transferrin, hemopexin, fibrinogen, the immunolobulins such as IgG, IgM, IgA, IgD, and IgE, and their fragments, e.g., Fc and Fab', complement factors, prolactin, blood clotting factors such as fibrinogen, thrombin and so forth, insulin, melanotropin, somatotropin, thyrotropin, follicle stimulating hormone, leutinizing hormone, gonadotropin, human chorionic gonadotropin, thyroid stimulating hormone, placental lactogen, intrinsic factor, transcobalamin, serum enzymes such as alkaline phosphatase, lactic dehydrogenase, amylase, lipase, phosphatases, cholinesterase, glutamic oxaloacetic transaminase, glutamic pyruvic transaminase, and uropepsin, endorphins, enkephalins, protamine, tissue antigens, bacterial antigens, viral antigens such as hepatitis associated antigens (e.g., hepatitis-B surface antigen, hepatitis-B core antigen, and hepatitis-B e antigen, and tumor markers (e.g., carcinoembryonic antigen, alpha fetoprotein, prostatic acid phosphatase, prostatic specific antigen, neuron specific enolase, estrogen receptor, cancer antigen 125, cancer antigen 19-9, and the like).

Representative hapten analytes include the general classes of drugs, metabolites, hormones, vitamins, toxins and the like organic compounds. Haptenic hormones include thyroxine and triiodothyronine. Vitamins include vitamins A, B, e.g., thiamine, $B_{12}$, C, D, E and K, and folic acid. Drugs include antibiotics such as aminoglycosides, e.g., gentamicin, tobramycin, amikacin, sisomicin, kanamycin, and netilmicin, penicillin, tetracycline, terramycine, chloromycetin, and actinomycetin; nucleosides and nucleotides such as adenosine diphosphate (ADP) adenosine triphosphate (ATP), flavin mononucleotide (FMN), nicotinamide adenine dinucleotide (NAD) and its phosphate derivative (NADP), thymidine, guanosine and adenosine; prostaglandins; steroids such as the estrogens, e.g., estriol and estradiol, sterogens, androgens, digoxin, digitoxigenin, digitoxin, digoxigenin, 12-0-acetyldigoxigenin, and adrenocortical steroids; and others such as phenobarbital, phenytoin, primidone, ethosuximide, carbamazepine, valproate, theophylline, caffeine, propranolol, procainamide, quinidine, amitryptiline, cortisol, desipramine, disopyramide, doxepin, doxorubicin, nortryptiline, methotrexate, imipramine, lidocaine, procainamide, N-acetylprocainamide, amphetamines, catecholamines, and antihistamines. Toxins include acetyl T-2 toxin, aflatoxin, cholera toxin, citrinin, cytochalasins, staphylococcal enterotoxin B, HT-2 toxin, and the like.

The liquid test mixture will be formed in such a manner that microparticles will become aggregated as a direct or inverse function of the presence or amount of the ligand of interest. Typically, this will be accomplished by employing microparticles that are coated with the ligand or with a binding analog or binding partner of the ligand. A binding analog of the ligand can be any substance which is recognized and bound by the binding partner sufficiently for the assay to be meaningful. Such binding analogs will often be chemical derivatives or fragments or precursors of the ligand. Coating of the microparticles with the ligand, binding analog, or binding partner can involve the formation of covalent bonds or noncovalent chemical binding such as adsorption by hydrophobic or ionic bonding, and the like. The choice of a particular means for coating the microparticles will be essentially one of convenience and optimization for the assay.

The microparticles can be selected from any of a wide variety of substances capable of aggregating and thereby altering the effective dielectric constant of the liquid test mixture. In their non-aggregated state, the microparticles will exhibit an effective dielectric constant that is significantly different from an analytical standpoint from the dielectric constant of the liquid test mixture. Generally, the microparticles will have an average diameter of between about 20 and about 80 nm, preferably between about 30 and about 50 nm. The microparticles can be composed of a variety of materials, preferably non-magnetic, such as polystyrene, polyamide, or other plastic microbeads or latexes, ceramic particles, and metal sols. Metal sols are particularly preferred. Such microparticles are prepared by methods well known in the art. For example, gold sols typically are prepared by oxidizing aqueous aurate chloride solutions. A typical preparation would entail oxidizing a 0.1% (w/w) tetrachloroaurate solution with 0.2% (w/w) sodium citrate for 30 minutes at 100°C. Gold sols are particularly advantageous.

Microparticle aggregation is determined in accordance with the present invention by measuring the change in a dielectric property of the liquid test mixture in the microwave region, which falls between about $5 \times 10^8$ and about $3 \times 10^{11}$ hertz (Hz), by means of microwave spectroscopy. Any means of measuring changes in absorption of energy in the microwave region can be applied for this purpose. The use of resonance circuits are particularly preferred. Microwave resonance circuits can be in a variety of forms. Examples of resonant circuits are cavities, or their dual-dielectric resonators; in general any geometric structure that allows the conversion of electrical to magnetic stored energy at that frequency of oscillation can be termed to be in resonance with the exciting electromagnetic radiation. In the visible range of the spectrum a familiar example would be a laser cavity. Resonant circuits therefore also include geometric structures such as striplines which are prepared like the common electronic printed circuits. In the microwave range, different substrate materials are used such as ceramics but the photographic and etching techniques or silk screening for the geometric patterns is very similar. The use of microwave stripline resonance devices are particularly preferred.

Changes in the dielectric properties of the liquid test medium due to aggregation of the microparticles can be measured by any well known technique such as measurement of propagation constant, reflectance spectroscopy, and time domain spectroscopy.

The aggregation of microparticles can be designed to occur in a liquid solution or suspension, or at the surface of a solid substrate. In the former case, the present method preferable involves the steps of:

(a) forming a liquid test mixture containing said test sample and microparticles which are coated with said ligand or a binding analog or a binding partner thereof, and if said microparticles are coated with said ligand or binding analog thereof, then the liquid mixture is formed to additionally contain a diffusible form of a binding partner of the ligand, the coated microparticles having an effective dielectric constant that is significantly different from the dielectric constant of the test mixture in the absence of ligand in the test sample, and

(b) measuring any change in dielectric constant of the test mixture in the microwave region as a function of the amount or presence of ligand in the test mixture by means of microwave resonance spectroscopy.

One example of such a liquid aggregation method is the determination of a multivalent ligand by direct aggregation of microparticles coated with binding partner. By multivalent is intended the property of being capable of binding to more than one binding partner, therefore permitting the formation of networks comprising multiple ligands bound to multiple microparticles. The extent of aggregation of the microparticles is thus a direct function of the amount of ligand present.

Another example is the determination of a univalent ligand, in which case a diffusible, multivalent form of the ligand or a binding analog thereof is added along with the microparticles coated with binding partner. Such multivalent reagent serves to aggregate the microparticles in the absence of ligand. Thus, since ligand will compete with the multivalent reagent for binding to the microparticles, the extent of aggregation will be inversely related to the amount of ligand present.

A further example of a liquid aggregation method is the determination of a multivalent or univalent ligand by addition of (i) microparticles coated with the ligand or a binding analog thereof and (ii) a diffusible form of a binding partner for the ligand. Here again ligand will compete with the microparticle bound ligand or analog for binding with the diffusible binding partner, the result being an inverse relationship between the extent of resulting aggregation and the amount of ligand in the test mixture.

The present method can also be accomplished using a solid substrate device, in which case, the method will generally comprise the steps of:

(a) forming a liquid test mixture containing said test sample and microparticles which are coated with said ligand or a binding analog or a binding partner thereof, said test mixture being in contact with a solid substrate to which is bound a binding partner of said ligand, the coated microparticles having an effective dielectric constant that is significantly different from the dielectric constant of the test mixture in the absence of ligand in the test sample, and

(b) measuring any change in dielectric constant of the test mixture in the microwave region at the surface of said solid substrate as a function of the amount or presence of ligand in the test mixture. Optionally, the liquid mixture can be removed from contact with the solid substrate prior to the measurement step, and further such surface can be optionally washed in order to remove microparticles that might be non-specifically bound thereto.

In one form of this method, multivalent ligands are determined by using solid substrate and microparticles that are both coated with binding partners, usually directed to mutually distinct binding sites on the ligand. Such a sandwich-type format results in association of the microparticles with the substrate surface in direct relation to the amount of ligand present. In a competitive format, the microparticles are coated with the ligand or a binding analog thereof. Thus, ligand in the test mixture competes with the microparticle reagent for binding to the binding partner on the substrate surface and the degree of association of the microparticles with the surface is therefore inversely related to the amount ligand present.

The present invention will now be illustrated, but is not intended to be limited by, the following examples.

EXAMPLE 1

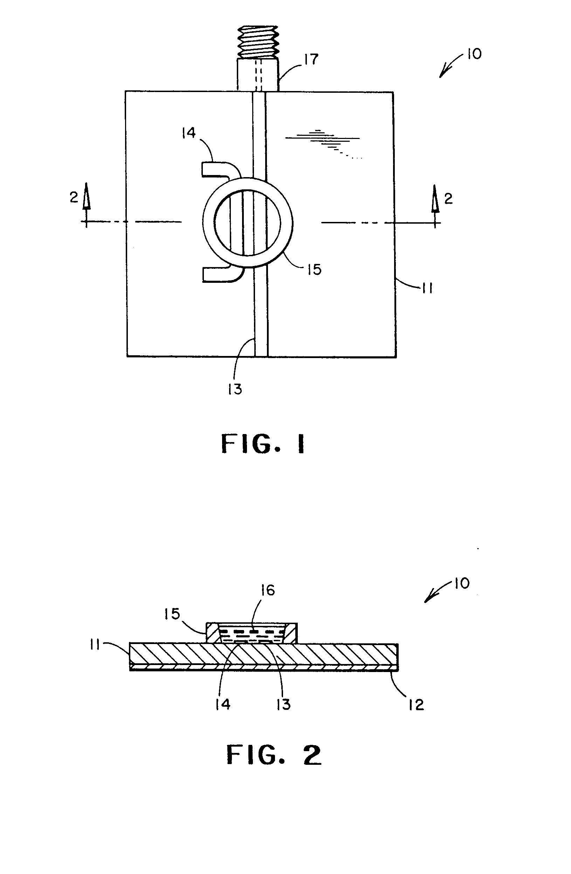

With reference to Figs. 1 and 2 of the drawings, a resonant circuit (10) was fabricated on a material obtained from Murata-Erie (State College, PA, USA) with a dielectric constant of 80 (11) and disposed on a ground plane of silver (12). The shape was in the form of a directional coupler, i.e., a silver paste transmission line (13) with a U-shaped silver paste section (14) closely spaced (0.002 inches) next to it. An epoxy well (15) was made on top of this gap by using a polyethylene pipette tip as a form. The well could contain a 90

microliter ($\mu$L) sample volume (16), but only 30 $\mu$L was necessary. This resonant circuit with epoxy sample well was mounted on a type N coaxial to microstripline adapter (17). The assembly was then analyzed on a Hewlett-Packard 8753A network analyzer (Hewlett-Packard, Palo Alto, CA, USA). The reflectance spectrum was recorded and a minimum was found at 2.6 Gigahertz (GHz, - $10^9$ cycles per second), which changed in its position when samples were presented to the resonant circuit.

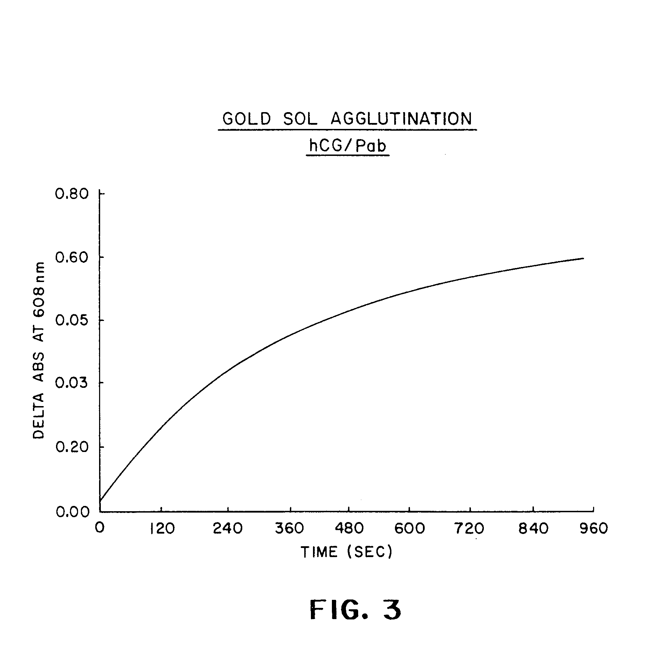

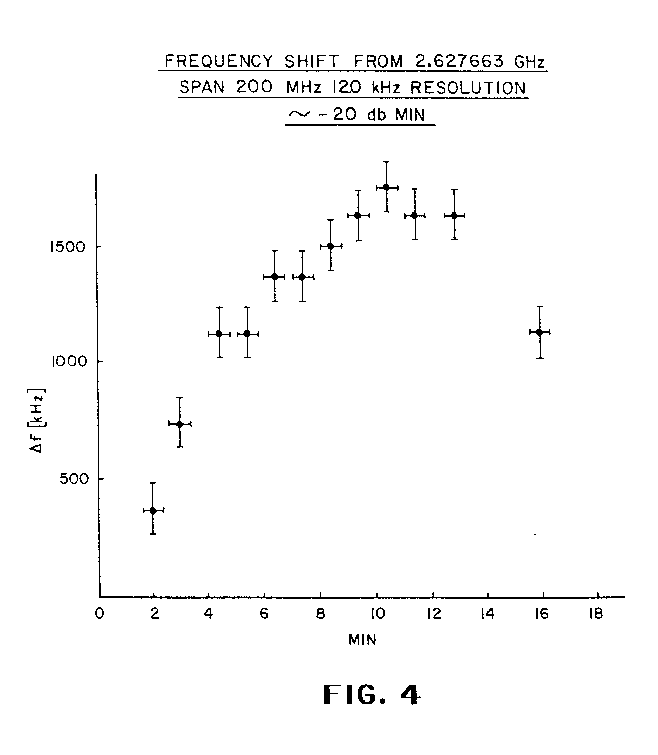

A gold sol was coated with human chorionic gonadotropin (hCG) protein by the following procedure. A 100 $\mu$l of an aqueous hCG solution (250 $\mu$g of total protein) was rapidly added to a vigorously stirred 10 ml solution of the gold sol. After 10 minutes, 0.5 ml of 1% (w/w) polyethylene glycol (20,000 MW) (PEG) was added. After an additional 10 minutes, 1 ml of 10% bovine serum albumin (BSA) in 2mM, pH 9 sodium borate buffer was added. The suspension was centrifuged at 10,000 rpm and the supernatant removed. Six washes with 1% BSA and 0.05 PEG in borate buffer were performed. The protein coated gold sol was then reconstituted with water to the desired concentration. The particles were 40 nanometers (nm) diameter as measured on a Coulter light scattering instrument. The suspending solution was phosphate buffered saline with 1% (w/v) bovine serum albumin and polyethylene glycol (MW 6,000, 1.5% w/v). When hCG polyclonal antiserum is added at a 1/100 dilution to the gold sol solution, an agglutination reaction occurs which was monitored by measuring absorbance at 608 nm versus time (Figure 3). The same system was also put into the sample well of the microwave resonant circuit and the frequency shift of the resonance was followed (Figure 4). A control solution with no antibody produced no frequency shift or absorbance change.

EXAMPLE 2

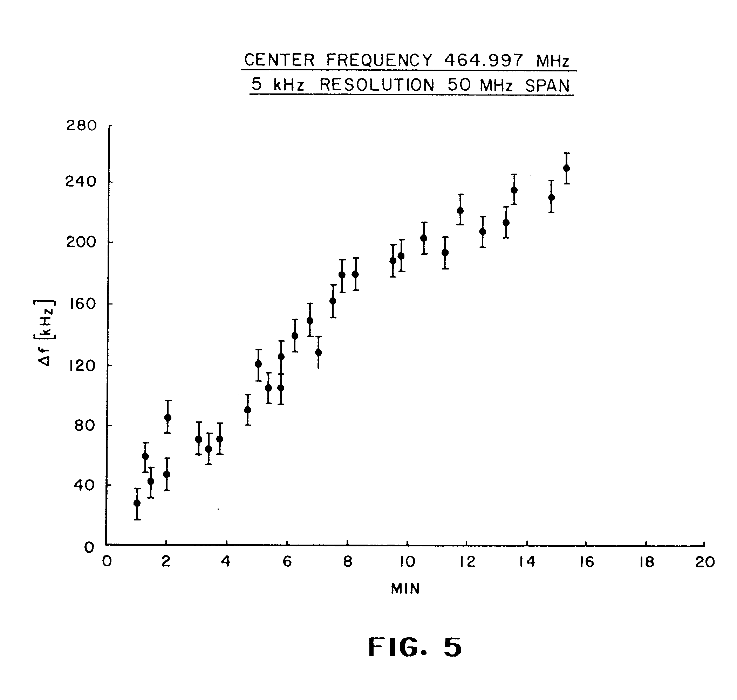

Another resonant circuit of different geometry was made. It consisted of three sections: capacitor-inductor-capacitor, all made in stripline configuration on dielectric material from Murata-Erie ($\epsilon' = 80$). An epoxy well was made on the second capacitor to receive a liquid sample of total volume 10 $\mu$L. The network analyzer (HP8753A) was used to measure a resonant frequency of 465 Megahertz (Mhz - $10^6$ cycles per second) in the presence of a sample. The same agglutination reaction as quoted in Example 1 was used in this resonant circuit. The center frequency was monitored during the reaction (Figure 5). A control solution produced a drift of small magnitude in the opposite direction. The experiment was repeated with similar results.

EXAMPLE 3

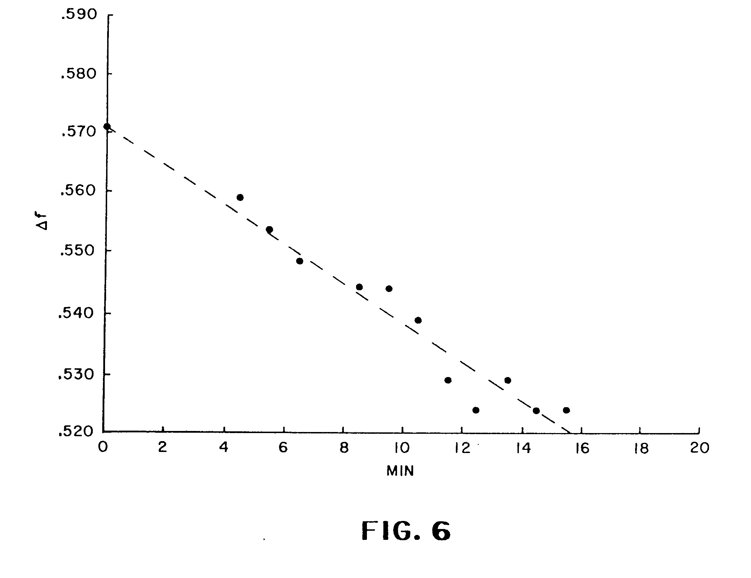

In order to improve sensitivity, a new circuit was designed where the resonant circuit formed the circuit element responsible for the frequency of oscillation of an active oscillator. A maximum output of energy would then occur at the frequency of oscillation and its accurate measurement would be less influenced by noise than a passive absorption type measurement. The circuit chosen was a dielectric resonator in the form of a cylinder (the dual of a microwave cavity) because of its high figure of merit (Q or sharpness of the spectral output). The sample was a model system of an agglutination, a chemically induced aggregation of gold sol particles. Gold sol of 40 nm diameter particles was stabilized by the addition of citrate. An agglutination was started by addition of pyridine (final concentration $10^{-5}$ M). The change in frequency of the oscillator was again monitored (Figure 6).

EXAMPLE 4

The dielectric resonator of Example 3 can be formed into a more convenient shape for sample presentation. The material can be made porous to such an extent that the sample can be imbibed by it. The resonance of the circuit would again shift due to the reaction proceeding in the liquid phase of the porous plug which is placed close to a selected spot of a transmission line forming the oscillating circuit.

The present invention has been particularly described and exemplified above. Clearly, many other variations and modifications of the invention can be made without departing from the spirit and scope hereof.

**Claims**

1. A method for determining a ligand in a test sample wherein a liquid test mixture is formed in which microparticles are aggregated as a direct or inverse function of the presence or amount of said ligand in said test sample, characterized in that the microparticles have an effective dielectric constant that is significantly different from the dielectric constant of the test mixture in the absence of ligand in the test sample, and changes in a dielectric property of the test mixture in the microwave region is measured as a function of the presence of ligand in the sample.

2. The method of claim 1 wherein the dielectric constant of the test mixture is measured by means of microwave resonance spectroscopy.

3. The method of any of claims 1 and 2 wherein the dielectric constant of the test mixture is measured with a microwave stripline resonance device.

4. The method of any of claims 1 - 3 wherein the microparticles are coated with said ligand or a binding analog or binding partner thereof.

5. The method of any of claims 1 - 4 wherein said aggregation occurs in a liquid solution or suspension.

6. The method of any of claims 1 - 5 wherein the microparticles are coated with said binding partner of the ligand and wherein the test mixture additionally contains a diffusible, multivalent form of said ligand or a binding analog thereof.

7. The method of any of claims 1 - 6 wherein the microparticles are coated with said ligand or a binding analog thereof and the test mixture additionally contains a diffusible form of said binding partner.

8. The method of any of claims 1 - 7 wherein the microparticles have an average diameter of between about 20 and about 80 nm.

9. The method of any of claims 1 - 8 wherein the microparticles are a metal sol, preferably a gold sol.

10. The method of any of claims 1 - 9 wherein said ligand is an antigen or hapten; an oligonucloetide or a polynucleotide; or a hormone, metabolite, or drug; and wherein said binding partner is, respectively, an antibody or a fragment thereof; a complementary oligonucleotide or polynucleotide; or a receptor.

**FIG. 1**

**FIG. 2**

GOLD SOL AGGLUTINATION
hCG/Pab

FIG. 3

FREQUENCY SHIFT FROM 2.627663 GHz
SPAN 200 MHz 120 kHz RESOLUTION
~ - 20 db MIN

FIG. 4

EP 0 519 250 A2

CENTER FREQUENCY 464.997 MHz
5 kHz RESOLUTION 50 MHz SPAN

FIG. 5

FIG. 6